# EUROPEAN PATENT APPLICATION

(11) **EP 4 696 132 A1**
(43) Date of publication of application: **18.02.2026**
(21) Application number: 24788642.7
(22) Date of filing: 03.04.2024
(51) Int. Cl.: A01H 3/00, A01H 6/54

(54) **METHOD FOR INTRODUCING MALE STERILITY OF TRUE DICOTYLEDONOUS PLANT**

(30) Priority: 12.04.2023 JP 2023064832
(71) Applicant: National University Corporation Tottori University, Tottori-shi Tottori 680-8550 (JP)
(72) Inventor: ISHII Takayoshi, Tottori-Shi, Tottori 680-8550 (JP); SEKIGUCHI Yuka, Tottori-Shi, Tottori 680-8550 (JP)
(74) Representative: Hoffmann Eitle
(86) International application number: PCT/JP2024/013763
(87) International publication number: WO 2024/214615

(57) **Abstract**

Provided is an agent for inducing male sterility in eudicot plants comprising trifluoromethanesulfonamide (TFMSA) as an active component. A method for inducing male sterility in a eudicot plant is also provided, the method comprising administering TFMSA to the eudicot plant.

## Description

### Technical Field

The present disclosure relates to a method for inducing male sterility in eudicot plants. The method may be useful, for example, in the breeding of agricultural crops and the efficient production of hybrid seeds.

### Background

Crossing is essential for plant breeding. Hybrid seeds produced by crossing play extremely important roles in the agricultural industry. In these contexts, crossing refers to the artificial pollination of one plant with another. For self-pollinating plants, where pollination occurs within a single plant, the crossing requires manipulations for emasculation, i.e. removal or sterilization of the stamens, in order to avoid the self-pollination. Conventional emasculation techniques include manual and physical removal of stamens, hot water emasculation, and the use of cytoplasmic male sterile lines. However, some of the major limitations associated with these techniques are that they are laborious and time-consuming and that they are available only in some suitable plants. For example, cytoplasmic male sterility depends on the presence of a specific genetic background, and hot water emasculation is available in grasses but it is impractical in legumes. Manual removal of stamens was used by Mendel in his famous crossing experiments, but long engagement in this labor is said to have caused deterioration of Mendel's eyesight (Chiharu NAKAMURA "Mendel Kai-Dai: The Story of the Priest Who Opened the Door to Genetics", Chapter 7, Intergenomics Study Group, Faculty of Agriculture, Kobe University, 2016). Therefore it is far from being an efficient technique, especially from an industrial point of view.

In monoecious plant species such as maize, controlling pollination is relatively easy because the flowers for the pistil and for the stamen are separate on the individual plant. In contrast, in legumes, for example, which have hermaphroditic and cleistogamous flowers, and which have the pistil and stamens aligned in the same position, the pollination can occur within each flower even before the flower opens, almost ensuring the self-pollination. Control of pollination in these species is particularly difficult, and traditionally the inefficient method of manual removal of stamens has been practically the only available option.

Legumes are consumed as a staple food worldwide and are also highly valued as animal feed. Cowpea, in particular, is an agricultural product that can be grown in arid zones and is potentially key to improving food security in arid zones such as sub-Saharan Africa. The use of soybeans and the like in the developing industry of meat alternatives has also become increasingly important in recent years. The development of efficient methods to emasculate and control pollination in legumes could represent a breakthrough in the legume breeding and have the potential to have a positive impact on the global food insecurity. There is a long-felt but unsolved need to efficiently induce male sterility, especially in legumes due to the current limited availability of genetic tools for male sterilization in legumes such as cowpea and the difficulty to physically control self-pollination as mentioned above.

Methods for inducing male sterility by using chemical agents (chemical emasculation methods) are also known for some agricultural crops, including rice, wheat, maize, cotton, and sunflower (Non-Patent Document 1). The chemical emasculation methods have the advantage of being able to treat the plants on a large scale and emasculate a large number of plants in a short period of time. The chemicals which are the active agents used in the chemical emasculation methods are called CHAs (chemical hybridizing agents). Development of CHAs has a long history, starting as early as the 1950s. As a result, quite a few types of CHAs have become known. However, each CHA is usually discovered and described for a specific plant group, and it is not easy to identify a substance that can effectively function as a CHA for a plant species of interest from among known CHAs. For example, Non-Patent Document 1 describes that maleic hydrazide (MH), which was first found to have a potential to induce male sterility in maize in 1950, has not proved useful as a CHA on any crop, in spite of the ensuing enthusiasm for testing MH on many crop species, primarily because it is not adequately male/female selective.

As can be seen in the above description of Non-Patent Document 1, most of the substances that are potential CHA candidates have some forms of toxicity or inhibitory activity on plant tissues. Therefore, they often cause not only male sterility but also female sterility (that is, have low male/female selectivity), or adversely affect vital processes such as germination of the plants. Since the diverse plant groups present in nature and used in agricultural industry have very different structures and mechanisms for reproduction, it is difficult to find effective CHAs that can exert male/female selectivity for each plant.

Patent Document 1 describes the use of a sulfonylurea derivative, which characteristically has a urea group and a hetero-six-membered ring, as a gametocide.

### Citation List

### Non-Patent Documents

Non-Patent Document 1: McRae, D. (1985) Plant Breed. Rev. 3: 169-192.

### Patent Documents

Patent Document 1: JP S64-68305 A

### Summary of the Invention

The present inventors have discovered that trifluoromethanesulfonamide (TFMSA) provides an effective male sterility inducer, or CHA, in a wide range of eudicot plants. TFMSA could induce male sterility in leguminous eudicots without showing substantial phytotoxicity or female sterility. Similar results were also obtained in other eudicot plants. Loussaert D (2004) Sexual Plant Reproduction 16(6): 299-307 had reported that TFMSA acted as a male fertility inhibitor in maize, a monocotyledonous Poaceae. Hodnett GL, Rooney WL (2018) Canadian Journal of Plant Science 98(5): 1102-1108 had reported that TFMSA could also induce male sterility in sorghum (also known as broomcorn, Guinea corn, etc.), a Poaceae closely related to maize. This paper stated that TFMSA might have applications in sorghum given the similarities between sorghum and corn. However, the present finding that TFMSA can provide an effective male sterility agent in eudicots is a surprising and significant discovery, as eudicot plants, which diverged from the group including monocots about 123 million to 147 million years ago (Bell et al. (2010) American Journal of Botany 97(8): 1296-1303), are remarkably different from Poaceae in phylogenetic, developmental, plant structural, and molecular biological (e.g., with respect to receptors and transporters) aspects.

For example, monocot and non-eudicot plants have "monocolpate pollen grains" which have only one aperture for extending the pollen tube (germination pore), whereas eudicot plants have evolved independently to produce tricolpate pollen grains, that is, three-apertured pollens. Kurata (Eco-Habitat : JISE Research, 26(1):53-66, 2020) describes this point as follows: "It is advantageous to have more germination pores. For example, it is difficult for monocolpate pollen grains to germinate/extend the pollen tube when the surface attached to the stigma is on the opposite side of the germination pore. In contrast, if there are multiple pores, pollen tubes can be extended from any of the multiple pores. This must have been an advantageous strategy for reproduction acquired during the evolution." It representatively illustrates that the pollen of eudicots cannot be equated with the pollen of Poaceae in phylogenetic, structural, developmental and other aspects, as discussed above, and supports the non-obviousness of the invention disclosed herein. Furthermore, it is well known that there is a great diversity within the eudicots themselves, and it was therefore surprising that TFMSA could act as a male sterility agent in various distantly related eudicots.

Proline is known to be a key amino acid required for pollen development in plants. For example, Schwacke et al. (1999) Plant Cell, Vol. 11, 377-391 described that proline accumulated in tomato pollen to represent more than 70% of the total free amino acid content. The work of Loussaert (supra) teaches that inhibition of the proline transport, which is required for proline accumulation, is the mechanism of action of TFMSA as a male sterility inducer in maize. Loussaert showed experimentally that TFMSA reduced proline accumulation in the maize anthers (the pollen-producing organs) in a dose-dependent manner, that the change in proline levels was the earliest detectable change in metabolites observed after the application of TFMSA and before any symptoms of pollen failure were evident, and that application of TFMSA actually reduced proline transport in the anthers. Loussaert further teaches as follows: "It is inferred that TFMSA induces male sterility by interfering with the transport of proline from the site of synthesis to the site of accumulation, resulting in feedback inhibition of proline biosynthesis, ultimately starving the developing anther of proline." Hodnett et al. (supra) also teaches that "TFMSA may induce male sterility in maize and sorghum by reducing anther proline in pollen."

Schwacke et al. (supra) have identified a proline transporter protein, LeProT1 (GenBank accession number AF014808), which is specifically and predominantly expressed in stamens/anthers/pollen of tomato (a eudicot plant). The most similar proline transporter homolog in maize, sequence-wise, appears to be XP_008651898, but still the amino acid sequence identity of this maize homolog to the tomato homolog is only 61% whereas the maize homolog shares a high amino acid sequence identity of about 92% with the most similar sorghum homolog. As expressed in the following statement by Hodnett et al.: "Given the interest in temporal sterility in sorghum systems and the similarities between sorghum and corn, there is a possibility that TFMSA or a sister compound might have applications in sorghum," it was scientifically reasonable to apply TFMSA to sorghum, a close relative of maize, to induce male sterility. However, as Loussaert had taught that inhibition of proline transport was the mechanism of action of TFMSA in maize, it was not reasonable to infer that TFMSA might have an effect of male sterilization in eudicots, which are different from maize in the structures of the proline transporters as well as in the organ structures around the pollen.

The present disclosure includes the following embodiments.
[1] An agent for inducing male sterility in eudicot plants, comprising trifluoromethanesulfonamide (TFMSA) as an active component.
[2] The agent for inducing male sterility according to [1], wherein the eudicot plants are of the family Fabaceae, the family Brassicaceae, or the family Solanaceae.
[3] The agent for inducing male sterility according to [1], wherein the eudicot plants are of the family Fabaceae.
[4] The agent for inducing male sterility according to [3], wherein the plants of the family Fabaceae are cowpeas.
[5] The agent for inducing male sterility according to any of [1] to [4], wherein the agent is in the form of an aqueous solution.
[6] The agent for inducing male sterility according to any of [1] to [5], wherein the agent is administered via the root of the plant.
[7] A method for inducing male sterility in a eudicot plant, comprising administering trifluoromethanesulfonamide (TFMSA) to the eudicot plant.
[8] The method according to [7], wherein the eudicot plant is of the family Fabaceae, the family Brassicaceae, or the family Solanaceae.
[9] The method according to [7], wherein the eudicot plant is of the family Fabaceae.
[10] The method according to [9], wherein the plant of the family Fabaceae is a cowpea.
[11] The method according to any of [7] to [10], wherein the TFMSA is administered in the form of an aqueous solution.
[12] The method according to any of [7] to [11], wherein the TFMSA is administered via the root of the plant.

### Brief Descriptions of the Figures

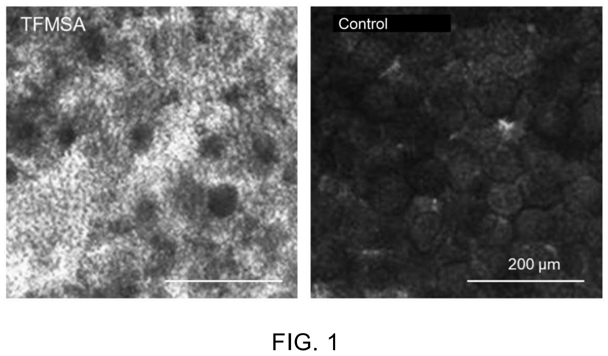

[Figure 1] FIG. 1 provides photomicrographs showing Alexander staining of the pollen, collected 7 days after the treatment, of the cowpea plants treated with the TFMSA dose of 30 mg (left, "TFMSA") or 0 mg (right, "Control") per plant.

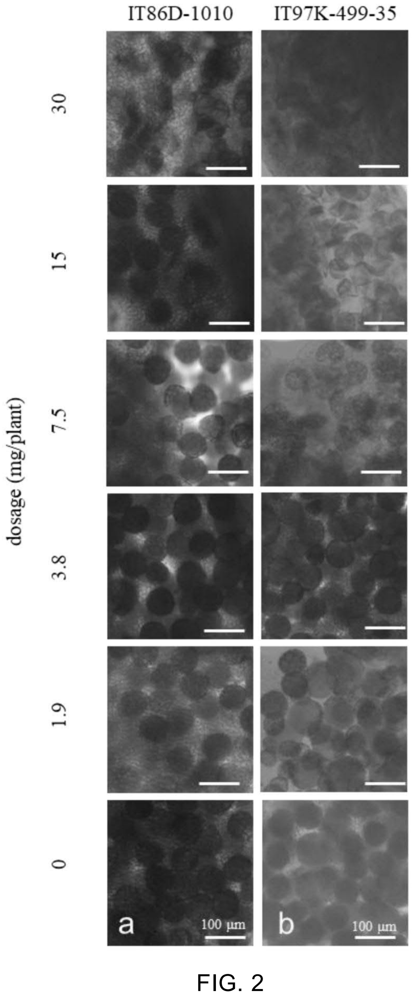

[Figure 2] FIG. 2 provides photomicrographs comparing Alexander staining of anthers of the cowpea plants treated with different doses of TFMSA per plant as indicated by the numbers (in mg) on the far left. The panels (a) represent strain IT86D-1010 and the panels (b) represent strain IT97K-499-35.

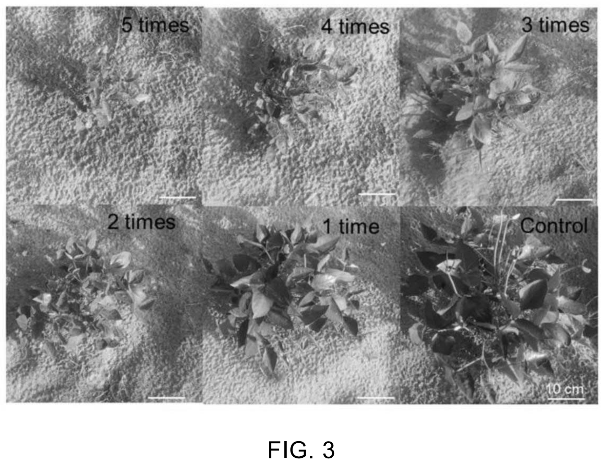

[Figure 3] FIG. 3 shows the results of the experiment showing that suppression of cowpea growth increases as the number of 30 mg TFMSA treatments increases. "5 times" indicates the plant treated with TFMSA five times at one-week intervals starting 5 weeks before anthesis, and "1 time" indicates the plant treated with TFMSA only once, one week before anthesis.

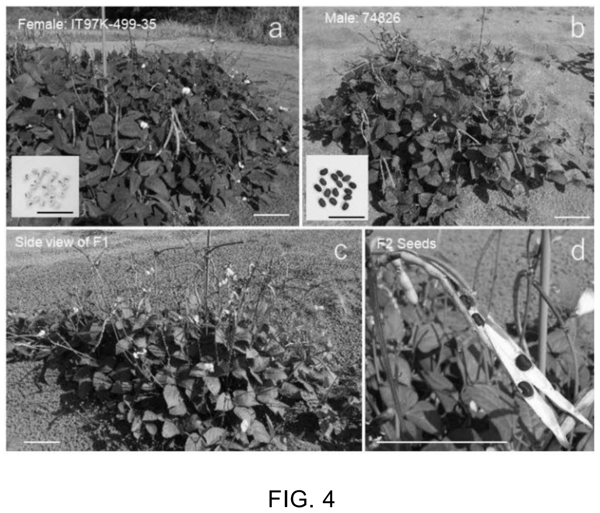

[Figure 4] FIG. 4 (a) is a photograph of the cowpea strain IT97K-499-35 with white seed coat. This strain was made male sterile by TFMSA treatment and used as a female parent for artificial pollination. (b) is a photograph of the cowpea strain 74826 with brown seed coat. This strain was not TFMSA treated and was used as a male parent to provide pollen for artificial pollination. The insets in (a, b) show photographs of the respective seeds. (c) shows IT97K-499-35 × 74826 hybrid F1 plants. (d) shows F2 seeds from the plant shown in (c), all of which have black seed coats. The white scale bar represents 5 cm and the black scale bar represents 3 cm.

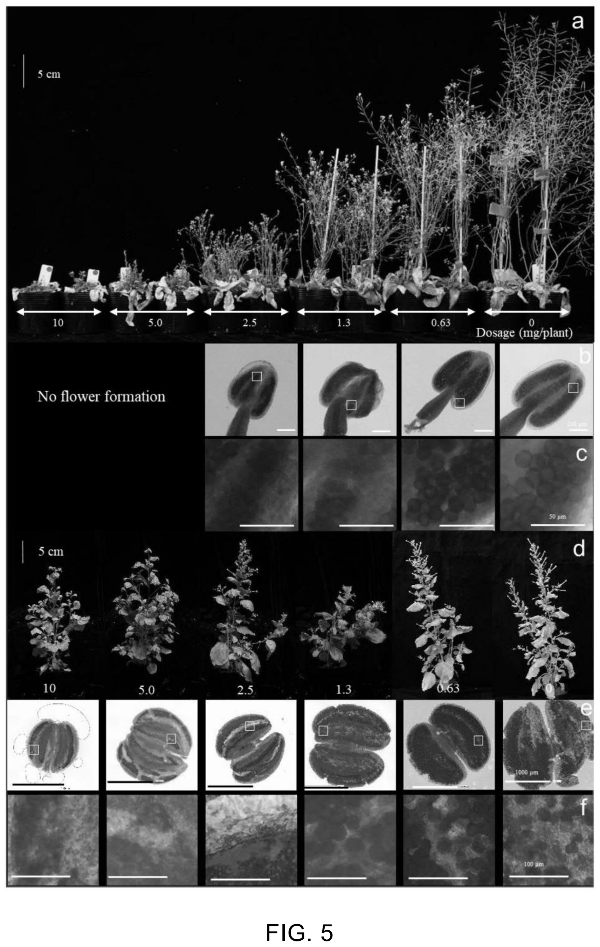

[Figure 5] FIG. 5 (a) shows the Arabidopsis plants 21 days after the second TFMSA treatments at different doses. The control plant treated with 0 mg dose is on the far right, and the plant treated with 10 mg dose is on the far left. (b) shows Alexander staining of the anthers of the plants in (a), 7 days after the second TFMSA treatments. (c) provides enlarged views of parts of (b). (d) similarly shows the tobacco plants 21 days after the second TFMSA treatments at different doses. (e) shows Alexander staining of the anthers of the plants shown in (d), 7 days after the second TFMSA treatments. (f) provides enlarged views of parts of (e).

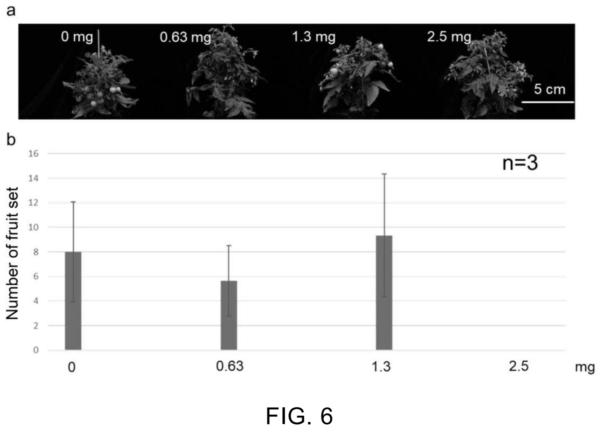

[Figure 6] FIG. 6 (a) shows photographs of Micro-Tom tomato, a Solanaceae, at fruiting stage after being treated with TFMSA doses of 0, 0.63, 1.3, or 2.5 mg per plant. (b) shows the graphs which correspond to the plants in (a) and summarize the numbers of fruit set. The number of fruit set decreased depending on the concentrations and no fruiting occurred in the plants treated with the 2.5 mg dose.

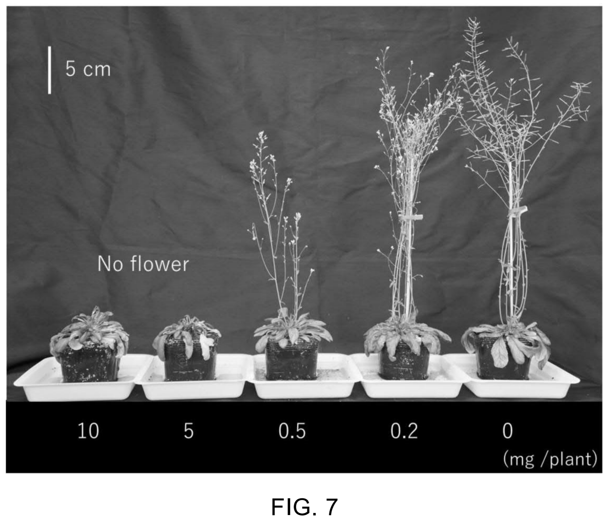

[Figure 7] FIG. 7 shows the application of solutions comprising different doses of dissolved TFMSA to Arabidopsis plants via roots. The control plant treated with 0 mg dose is shown on the far right, and the plants treated with different doses of TFMSA between 0.2 mg and 10 mg per plant are shown to the left. When 0.5 mg TFMSA was absorbed via roots, sufficient male sterility occurred and no seeds were formed.

### Detailed Description of the Invention

In one aspect, the present disclosure provides an agent for inducing male sterility in eudicot plants, comprising trifluoromethanesulfonamide (TFMSA).

The structural formula of trifluoromethanesulfonamide (TFMSA) is shown below.

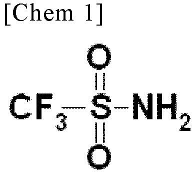

The eudicots in the present embodiments can be for example plants belonging to the core eudicots, and more specific examples include, but are not limited to, Fabidae, such as Fabales, or Malvidae, such as Brassicales, or Lamiids, such as Solanales. Some specific but non-limiting examples of preferable eudicots are Fabaceae which are Fabales, Brassicaceae which are Brassicales, and Solanaceae which are Solanales.

The eudicots in the present embodiments can be, for example, Fabaceae, Brassicaceae, or Solanaceae. Fabaceae (legumes) are particularly preferred. There has been a long-felt but unsolved need to efficiently induce male sterility in legumes for which (ironically, because they are emblematic of Mendelian genetics) efficient pollination control has traditionally been difficult and practically effective CHAs have not been known. The present embodiments provide a novel solution in the legume family. Legume flowers can be characterized by the pistil and stamens being aligned in the same position. It was surprising that the chemical TFMSA, administered indiscriminately, was able to act selectively on the male organs versus the female organs which are in such physical proximity. Non-limiting examples of Fabaceae plants include cowpea, soybean, fava bean, pea, kidney bean, chickpea, adzuki bean, lentil, mung bean, peanut, and rooibos. Other non-limiting examples of Fabaceae plants include Jack bean, African yum bean, Bambara groundnut, grass pea, Lablab, Ricebean, Sword bean, Velvet bean, Winged bean, and black gram.

The agent for inducing male sterility of the present embodiments can typically be present in the form of an aqueous solution. The term aqueous solution means a solution in which water is the primary solvent (accounting for 50% or more by weight of the solvent, for example 70% or more, 90% or more, or 100%). Relatively small amounts of other solvents may be added, such as, for example, ethanol. As with common agents used in the field of agriculture, the aqueous solution may comprise other components besides TFMSA, such as, for example, one or more of surfactants, thickeners, wetting agents, colorants, nutrients, and other active components. The other active components may be emasculation active components, or may have activity not directly related to emasculation, such as insecticidal, bactericidal or fungicidal activity. The agents for inducing male sterility according to certain embodiments may be provided in formulations other than aqueous solutions, such as, for example, powders, granules, microcapsules, tablets, emulsions, and the like. For example, use of a powder form which is applied as-is, as well as use of granules which are dissolved in water immediately prior to application may be contemplated.

The concentration of the active component TFMSA in the agent for inducing male sterility (e.g., in the form of an aqueous solution) may be, for example, 100 to 2000 mg/l or 500 to 1200 mg/l, but those skilled in the art may adjust the concentration appropriately in view of the present disclosure and ordinary knowledge and depending on the particular applications.

In another aspect, which has correspondence to the above-described aspect, the present disclosure provides a method for inducing male sterility in a eudicot plant, the method comprising administering TFMSA to the eudicot plant. The term administering as used in the present disclosure means making the active component TFMSA to adhere to or be absorbed by the eudicot plant (e.g. at its leaves or roots). It is to be understood that this TFMSA may be administered in the form of the agent for inducing male sterility described above. In other words, the present disclosure provides TFMSA, or a male sterility-inducing agent or composition comprising it, for use of inducing male sterility in eudicot plants. The aspect of the agent for inducing male sterility and the aspect of the method in the present disclosure have the corresponding relationship. Therefore, the descriptions provided above regarding the subject eudicot plants, the TFMSA formulations, etc., may also be applied to the method embodiments. In the following descriptions, any reference to TFMSA may be alternatively understood as a reference to a TFMSA-containing composition or male sterility inducing agent.

A person skilled in the art can predict with a certain degree of accuracy the time of flowering or when normal pollination will occur, based on the timing of sowing, cultivation conditions and empirical experience. In the present embodiments, TFMSA may be administered prior to anthesis, preferably 5-20 days prior to anthesis. TFMSA may be administered, after the confirmation of anthesis and removal of the flowers and the sheaths, to affect the subsequent flowers. Male fertility appeared to be restored after two or more months of the TFMSA administration. In that sense, the induction of male sterility by TFMSA for eudicots may be reversible. It has been found that TFMSA can have growth inhibitory effects to some extent in eudicot plants. Those skilled in the art with reference to this disclosure will be able to adjust the dose of TFMSA per administration depending on the type of plant or the size of the biomass. The dose may typically be in the range of 1.5 to 50 mg per plant or, for example 0.5 to 3 mg for small plants. In the case of cowpea, for example, 20 to 40 mg, particularly 30 mg, per plant is preferred. Multiple doses may be administered, in which case the interval between one dose and the next may be for example 5 to 10 days.

In an embodiment, the present disclosure provides a schedule for inducing male sterility in cowpea. The method according to this schedule comprises administering, prior to anthesis, 2 doses of 20 to 40 mg (e.g., 30 mg) TFMSA per cowpea plant at an interval of 5 to 10 days (e.g., 7 days). The second dose may be 5 to 10 days prior to anthesis. This dosing schedule can ensure complete male sterility while maintaining female fertility and sufficiently avoiding the suppression of plant growth, regardless of cowpea strain.

TFMSA is typically administered to the above-ground parts of the plant, including the leaves. For example, it is preferable to perform the administration such that all leaves found at the time of administration receive on their surfaces TFMSA or a solution containing it. It is not excluded that TFMSA applied to the soil may be absorbed through the roots and act on the above-ground parts, as illustrated in the Examples below. The effectiveness of TFMSA administration through the roots has been confirmed not only in dicotyledonous plants but also in monocotyledonous plants such as pearl millet. Accordingly, the present disclosure in one aspect provides a method for inducing male sterility in a plant, comprising making the plant absorb TFMSA through the roots, and also provides a male sterility-inducing agent comprising TFMSA for use in such a method. The plant in this case may be a dicotyledonous plant, particularly a eudicotyledonous plant, or a monocotyledonous plant. Whether the location of the administration is above ground or below ground, suitable methods of administration may be appropriately determined by one skilled in the art, which may include, but are not limited to, spraying with a sprayer; sprinkling with a watering can, dropper, or the like; applying with a brush, sponge, or the like; adding to the nutrient solution; or applying a liquid formulation or a solid formulation such as a tablet, granules or the like onto the ground surface and/or into the ground.

### Examples

The invention will be more specifically described below with examples, but these examples merely describe typical experiments for illustrative purposes, and the present invention is not limited to these specific embodiments. For example, similar results can be obtained with eudicot plants other than the specific species described below.

### [Materials and Methods]

Between May and October, dozens of cowpea strains were sown simultaneously at the experimental farm of the Arid Land Research Center of Tottori University. Two additional strains (IT86D-1010 and IT97K-499-35) were grown in a greenhouse (26°C for 16 h day/18°C for 8 h night). Cowpea (*Vigna unguiculata* (L.) Walp.) is a eudicot plant of the family Fabaceae.

Trifluoromethanesulfonamide (TFMSA; Tokyo Chemical Industry Co., Ltd.) was dissolved in an aqueous solution containing Applauch BI (0.1% v/v, Maruwa Biochemical Co., Ltd.) which is a commercial agrochemical spreader (polyoxyethylene hexitane fatty acid ester surfactant). TFMSA solutions were thus prepared at different concentrations between 0 and 1000 mg/l (w/v). 0 mg/l was the control. The TFMSA solutions were sprayed on cowpeas once a week, starting 1 to 5 weeks before anthesis. It was confirmed that surfaces of all leaves were covered with the solution.

For the detailed analysis of pollen fertility, anthers were collected 1 week after the last TFMSA treatment and approximately 24 h before anthesis, fixed in 6:3:1 (v/v/v) ethanol/chloroform/glacial acetic acid at room temperature for 3 days, and then subjected to Alexander staining (Alexander M (1969) Stain Technology. 44(3): 117-122). The Alexander staining solution was prepared with the following composition: 10 ml 95% ethanol, 1 ml malachite green (1% solution in 95% ethanol), 50 ml distilled water, 25 ml glycerol, 5 ml acid fuchsin (1% aqueous solution), 0.5 ml orange G (1% aqueous solution), 4 ml glacial acetic acid. The fixed anthers were added to a 1.5 ml tube containing 200 µl Alexander staining solution and placed on a heat block at 95°C for 5 min. The anthers were then transferred to a glass slide, and microscopically imaged either by squashing the anthers between the glass slide and the coverslip or by cutting the anthers with a needle to disperse the pollen. With Alexander staining, fertile pollen grains are stained magenta and sterile pollen grains are stained blue. The "Pixel Classification + Object Classification" workflow of the machine learning software "ilastik" (Berg S, et al (2019) Nature Methods 16(12): 1226-1232) was used to count the number of fertile and sterile pollen grains. For this purpose, one flower was collected from each individual plant, two anthers were cut from each flower, and the total numbers of fertile and sterile pollen grains in the two anthers were counted.

The following experiments were performed to confirm the female function. The strain IT97K-499-35 cowpeas (white seed coat) were treated twice with 30 mg TFMSA per plant and then hand-pollinated with pollen from 29 other cowpea strains (brown seed coat). Two hybrid F1 seeds from each cross were sown on the farm and F2 seeds were also obtained. True hybridity, i.e. the presence of normal female function and absence of male function in the TFMSA-treated individuals, was confirmed by observing the seed coat color of the F2 seeds.

Essentially similar experiments to the cowpea experiments described above were performed in other eudicot plants, including diploid and tetraploid *Arabidopsis thaliana,* a member of the Brassicaceae family, and tobacco (*Nicotiana benthamiana*) and tomato (*Solanum lycopersicum*), both members of the Solanaceae family. Arabidopsis, tobacco, and tomato were treated with TFMSA when the first bud was observed. Diploid Arabidopsis plants were used for the experiments of TFMSA treatment from roots. Specifically, 100 ml of an aqueous solution containing 0 mg to 10 mg TFMSA with 0.1% Applauch BI per plant was poured once into a water tray, and the culture pots were placed on the water trays to allow the plants to grow while absorbing TFMSA from the roots.

### [Example 1]

The anthers of the cowpea treated twice with a TFMSA dose of 30 mg per plant showed a high degree of pollen sterility, i.e. male sterility, with predominantly blue-stained pollen grains as determined by Alexander staining. This result contrasted with the control cowpea pollen grains, which were generally stained magenta-red indicating fertility. The two types of pollen could also be distinguished by the fact that normal fertile pollen grains were generally round and large, whereas blue-stained sterile pollen grains were small and had relatively unclear outlines. Figure 1 shows the experimental results for the strain IT86D-1010. Different cowpea strains as well as lower dose TFMSA treatments were tested but occurrence of high degree of pollen sterility was observed in all strains tested (Figure 2). For example, significant pollen sterility was observed in the strain IT97K-499-35 at 7.5 mg TFMSA per plant, and large numbers of non-functional (blue-stained) pollen grains were observed at lower doses such as 1.9 mg and 3.8 mg.

Table 1 summarizes the results of detailed determination of fertile/sterile pollen grain counts after TFMSA treatments at different doses, twice for each dose, of the strain IT86D-1010 grown in the greenhouse. The percentages of fertile pollen grains in the TFMSA-treated cowpeas were significantly different from that in the control-treated cowpeas. The pollen sterility was 99% using a TFMSA treatment dose of 30 mg per plant.

**[Table 1]**

| TFMSA (mg per plant) | Number of plants investigated | Total number of pollen grains | Number of fertile pollen grains | Number of sterile pollen grains | Proportion of sterile pollen (%) | Significant difference from the Control |
|---|---|---|---|---|---|---|
| 0 (Control) | 2 | 519 | 412 | 107 | 19 | |
| 1.9 | 2 | 384 | 144 | 240 | 63 | ** |
| 3.8 | 3 | 738 | 303 | 435 | 59 | ** |
| 7.5 | 3 | 622 | 148 | 474 | 76 | ** |
| 15 | 3 | 846 | 274 | 572 | 68 | ** |
| 30 | 3 | 611 | 6 | 605 | 99 | ** |

| | | | | | | |
|---|---|---|---|---|---|---|
| ** A significant difference at 1% when compared with the Control group in the chi-square test. | | | | | | |

### [Example 2]

In the farm study using a single 30 mg TFMSA treatment per plant, complete pollen sterility was observed in 38 cowpea strains, 1 strain had partially functional pollen, and 3 strains did not appear to have been significantly affected. However, when these 3 strains were retested with 2 treatments per plant, male sterility could be induced.

### [Example 3]

When the farm-grown IT97K-499-35 strain cowpeas were treated before anthesis with a 30 mg dose of TFMSA per plant different numbers of times ranging from 0 to 5 times (at 1 week intervals), a delay in the growth could be observed as the number of treatments increased (Figure 3). The plants that received five TFMSA treatments, that is, every week for 5 weeks, were smaller than those that received only one TFMSA treatment in the last week of the 5 weeks. The average numbers of sheaths and seeds per plant were significantly lower in the treated plants compared to the untreated control plants. The seed number was significantly reduced following even one treatment, and hardly any seeds were produced in the plants that received 2 to 5 treatments.

### [Example 4]

When the cowpea strain IT97K-499-35 with white seed coat, which had been rendered male-sterile by two times of TFMSA treatments, was crossed with 29 strains of brown-seeded cowpeas which had not been TFMSA-treated, the former being the female parent and the latter male, F1 seeds were obtained which germinated normally to produce normal F2 seeds. These F2 seeds were black (Figure 4). The black seed coat indicates that the F1 plants are true hybrids (Herniter IA et al (2019) Frontiers in Plant Science 10). This demonstrates that an optimal TFMSA treatment, which causes pollen sterility, does not have a significant adverse effect on the female reproductive function.

### [Example 5]

Similar results can be observed in non-leguminous eudicots. For example, lack of functional pollen formation was observed after TFMSA treatment with a dose of 1.3 mg or more per plant in diploid Arabidopsis and 2.5 mg or more per plant in tobacco (Figure 5). In both Arabidopsis and tobacco, TFMSA treatments tended to suppress growth of the plants, as seen in the reduced plant heights. Arabidopsis is a plant naturally having a relatively small biomass, and it was more susceptible to growth suppression by the TFMSA treatment. At TFMSA doses of 5 mg or more per plant, the plant height was less than 1/4 of the control, and even the flower formation itself appeared to have been inhibited. However, branch number was not reduced in a TFMSA-dose-dependent manner in either species. Interestingly, the tetraploid Arabidopsis was more resistant to the growth suppression by TFMSA than the diploid counterpart, maintaining a high plant height at up to 10 mg, the maximum dose tested, and forming flowers at the doses of 5 to 10 mg (not shown). A similar effect of male sterility was also observed in tomato, which showed a concentration-dependent reduction in the number of fruit set and no fruit set at the dose of 2.5 mg (Figure 6).

While the foregoing paragraphs have described the examples of applying TFMSA solutions to the above-ground parts of the plants, especially leaves, it is also possible to induce male sterility by making the plant absorb TFMSA from the roots, for example by applying TFMSA to the soil or to the nutrient solution in a hydroponic culture. As shown in Figure 7, the treatment of diploid Arabidopsis with TFMSA exclusively through the roots resulted in a concentration-dependent reduction in the number of pods with seeds (this is in a self-pollination experiment). The treatment with 0.5 mg TFMSA caused significant male sterility showing a significantly reduced number of pods with seeds compared with the control, resulting in almost no seed set. Because male sterility caused by 0.5 mg TFMSA was still limited when it was applied to the leaves of the diploid Arabidopsis (Figure 5), the application through roots may be more efficient than the application through leaves. No flowers were formed when 5 mg or 10 mg TFMSA was applied through the roots. Although not shown, the efficacy of the TFMSA male sterility inducing agent administered through the roots was confirmed not only in dicots but also in monocots such as pearl millet.

Overall, the experiments in these model eudicot plants showed that although TFMSA can have side effects of growth inhibition dose-dependently, an optimal TFMSA dose that causes male sterility while avoiding severe growth inhibition and female sterility can be determined on a plant-by-plant basis.

In summary, these studies analyzed the utility of TFMSA as an effective male sterility inducer in eudicot plants including cowpea, Arabidopsis, tobacco and tomato. The lack of ability of the treated plants to form self-pollinated sheaths, detailed analysis of pollen sterility by Alexander staining in diverse genotypes and species, and evaluation of the effects of TFMSA administration under different conditions confirmed that this technology has robust efficacy in eudicot plants. For example, virtually complete male sterility was achieved with 30 mg×2 TFMSA treatments per plant in cowpea, and male sterility was also observed in other model eudicots, often at relatively low doses. Administration via roots was also effective. Although a clear tendency for TFMSA to have dose-dependent growth-retarding side effects in eudicots was observed, it seemed feasible to determine a suitable dose for a given eudicot species to induce male sterility while retaining female fertility and a sufficient degree of growth with reference to the examples disclosed above.

## Claims

1. An agent for inducing male sterility in eudicot plants, comprising trifluoromethanesulfonamide (TFMSA) as an active component.

2. The agent for inducing male sterility according to claim 1,
wherein the eudicot plants are of the family Fabaceae, the family Brassicaceae, or the family Solanaceae.

3. The agent for inducing male sterility according to claim 1,
wherein the eudicot plants are of the family Fabaceae.

4. The agent for inducing male sterility according to claim 3,
wherein the plants of the family Fabaceae are cowpeas.

5. The agent for inducing male sterility according to claim 1,
wherein the agent is in the form of an aqueous solution.

6. The agent for inducing male sterility according to claim 1,
wherein the agent is administered via the root of the plant.

7. A method for inducing male sterility in a eudicot plant,
comprising administering trifluoromethanesulfonamide (TFMSA) to the eudicot plant.

8. The method according to claim 7, wherein the eudicot plant is of the family Fabaceae, the family Brassicaceae, or the family Solanaceae.

9. The method according to claim 7, wherein the eudicot plant is of the family Fabaceae.

10. The method according to claim 9, wherein the plant of the family Fabaceae is a cowpea.

11. The method according to claim 7, wherein the TFMSA is administered in the form of an aqueous solution.

12. The method according to claim 7, wherein the TFMSA is administered via the root of the plant.
